# EUROPEAN PATENT APPLICATION

(11) **EP 1 988 156 A1**
(43) Date of publication of application: **05.11.2008**
(21) Application number: 07290543.3
(22) Date of filing: 30.04.2007
(51) Int. Cl.: C12N 5/00, C12N 13/00, C12M 1/42

(54) **Cell construct, its process of manufacture, as well as a device, implant and processes for the mechanical stimulation of cells in vitro and in vivo**

(71) Applicant: Brossel, Rémy, 13280 Raphèle les Arles (FR)
(72) Inventor: Brossel, Rémy, 13280 Raphele les Arles (FR)
(74) Representative: Touati, Catherine

(57) **Abstract**

The invention relates to a cell construct comprising cells in contact with at least one basement membrane and/or at least one stroma, wherein said basement membrane or stroma contains means for applying mechanical stimulation on said cells. The invention also relates to the process of manufacture of the cell construct and its use, as well as to a device, an implant and processes for the mechanical stimulation of cells *in vitro* and *in vivo.*

## Description

The present invention relates to cell cultures and cells that make up biological tissues. The invention focuses on cell stimulation resulting in changing cell phenotype as well as inducing or increasing cell differenciation or production of cell by-products. More specifically, this invention relates to the field of oncology, and to the treatment of cancer tumors, for example by changing the tumoral phenotype of cancer cells into a non-cancerous phenotype. The invention also relates to the field of fundamental research and particularly to laboratory models created by studying the change from a tumoral to a non cancerous phenotype.

More specifically, the present invention is directed to a cell construct comprising means for the mechanical stimulation of cells, which are themselves included into the cell construct. Interestingly, the mechanical stimulation of the invention is induced by an external field, said external field being *"wireless"* that is, having no direct contact with the cells. This invention also relates to a process of manufacture of the cell construct, to a device and to a process for the mechanical stimulation of cells found within the cell construct. Other objects of this invention are an implant as well as a process for inserting said implant into a targeted cell tissue for the mechanical stimulation of cells in vivo.

In recent years, the biomechanical dimension of tissue development has gradually been taken into account. Mechanical tensions *(tensions,* stresses, and *forces* are used interchangeably throughout the present application) within the cytoskeleton may impact cell form and function, for example growth, differenciation, apoptosis, angiogenesis, transcription, translation, signal transduction, gene expression and motility.

In fact, these internal tensions may influence tissue development, especially by acting on three key players of the cytoskeleton, namely actomyosin filaments (present in all cells), microtubules struts and intermediate filaments. In particular, mechanical internal tensions may be transmitted through cells (modifying their structures and activities) in the following manner: first, tension found in actomyosin filaments may be biochemically controled by the activation of Rho-kinase by Rho, a small G protein. The tension may then be exerted on the cellular membrane by the actomyosin filaments using integrins, which in turn, may increase isometric tension and promote "external fibers". Then, microtubules struts may buckle when compressed and intermediate filaments may transfer tension from microfilaments to microtubules. The tensional forces generated by the extracellular matrix/cytoskeleton may finally be transmitted to the nucleus. At low level of tension the actin lattice may be able to transfer the forces to the nucleus, independently of the cytoskeleton, when at high level intermediate and microfilaments may stiffen and anchor the nucleus (Maniotis AJ. et al., PNAS, 94:849, 1997).

Studies on the interactions (i.e. internal tensions) within the cytoskeleton, as well as the ones between the basement membrane and the cells have been numerous. However, the same cannot be said for interactions from and to the stroma.

In healthy tissues, the internal tensions between the stroma, basement membrane and cells, is in equilibrium (i.e. mechanical homeostasis). However, any disturbance on one of these three entities may affect the mechanics of the entire ensemble and may lead to the formation of tumor cells (i.e. cancer). In fact, by altering the internal cellular tension balance with external fields for example, one may remodel the pattern development of tissues. This alteration in the internal tension balance may change the shape of the entire cell and the nucleus, partly due to the hard wiring by cytoskeleton filaments (Maniotis AJ, above). Specifically, these changes in cell shape may produce a switch between different genetic programs, (post) transcriptional (including growth, death, differenciation) and may modify epigenetic expression, as shown in different lines of anchorage dependent epithelial cells (Chen CS et al., Science. 276:1425, 1997, Roskelley CD. et al., PNAS. 91:12378, 1994, Close M. et al., J. Cell Sci. 110:2861, 1997). Furthermore, extracellular matrix orientation may mediate tension dependent cell migration (Keller R. et al., Differentiation. 71:171, 2003), extracellular matrix rigidity may influence cell growth, death and differenciation (Yeung T, et al. Cell Motil. 60:24, 2005), and extracellular matrix compliance may influence cell contractility, Rho activity as well as ERK dependent growth (Wang HB. et al. Am. J. Cell Physiol. 279:c1345, 2000).

Some attempts have been made to stimulate cells and consequently affect their biological activities as well as the pattern development of tissues by applying external fields. For example, patent application WO 00/17322 (now abandoned) relates to a system for the stimulation of biological activies (e.g. gene expression, cell growth, cell differentiation, signal transduction, membrane permeability, cell division, and cell signalling) within cells by contacting the cells with an electroactive substrate. WO 00/17322 thus provides an electromagnetic stimulation coupled to an electromagnetic material (i.e. electroactive substrate) through which an electromagnetic field is applied to the cells.

Patent application WO 02/46365 provides a system comprising a flexible membrane, anchored to a device, and the supplying of cells to the flexible membrane. The membrane is said to be easily infiltrated with cells and is said to support cell attachment and growth. The cells will grow, expand and synthetize a matrix that will eventually transform into a connective tissue such as skin, bone, muscle, tendon, ligaments. In order to increase the rapidity at which the cells grow, they may be stressed by any suitable mechanical, electrical and/or biochemical means *in vitro.* However, WO 02/46365 does not include means embedded into a membrane and/or stroma for the application of a controlled, modulable and higly precise mechanical stimulation on said cells.

However, prior art devices do not permit a precise control of the changes occurring in the cells. Therefore, there is still a need for modifying the phenotype of cells, for example tumour cells, in a very guided and controlled manner, which in turn will permit one to predict the evolution in time of the phenotype and/or genotype changes of said cells.

Applicants thus propose means for changing the phenotype and/or genotype of cells or of tissues, by applying a controlled, modulable and higly precise mechanical stimulation. The inventors have found that, surprisingly, the use of a mechanical stimulation on the cells allows a much more precise control of their phenotype and/or genotype changes.

Moreover, Applicants also built a cell construct particularly suitable for the application of such mechanical stimulation, and found that, surprisingly, the cell construct of the invention is of particular application in the oncology field as well as in research and development.

As mentionned earlier, cancer implicates close contact and equilibrium between the internal tensions of three entities: the stroma, the basement membrane and the target cells. Although, these three entities communicate with each other by biochemical pathways (cytokines, growth factors, etc.), mechano-biochemical pathways (mechanotransductor) or by purely physical pathways, the application of various external forces to this "three-entity communicating unit" has yet to be fully searched.

In that respect, the cell construct of the present invention, comprising the previously mentioned "three-entity communicating unit", may be considered as representative of the *in vivo* situation and therefore allows for the *in vitro* study of controlled cell phenotype and/or genotype changes, from a cancerous phenotype to a non-cancerous phenotype and vice versa.

Furthermore, the Applicant has found that an implant as defined below may be inserted into a subject for in vivo cellular differenciation (i.e. phenotype changes for example) which may eventually lead to novel human treatment opportunities.

In the present invention, the words/expressions below are defined in the following manner:
- "basement membrane": the delicate layer of extracellular condensation of mucopolysaccharides and/or proteins underlying the epithelium of mucous membrane and secreting glands or the equivalent in the hematopoietic marrow which is a dense gluco-protein environment.
- "stroma": a framework comprising a cellular part made up of different types of cells, notably conjunctive cells and an acellular part usually called extrtacellular matrix, made up of a gel with numerous molecular constituents such as for example non fibrous collagen (contrary to the basement membrane) and other smaller molecules;
- "mucopolysaccharides": complex polysaccharides containing an amino group - they occur chiefly as components of connective tissue;
- "proteins": fibrous proteins, like in the basement membrane, or proteins in solution like in the extracellular membrane, for example collagen and/or fibronectin;
- "long-term culture initiating cells": equivalent to the stroma for the culture of hematopoietic stem cells;
- "hematopoietic stem cells": pluripotential stem cells which are capable of producing all lineages of white and red blood cells such as lymphocytes, granulocytes, platelets, erythrocytes, mast cells, macrophages and other cell types derived from monocytes, for example epithelial, hematopoietic, embryonic stem cells ;
- "skeletal muscle satellite stem cells": companions to voluntary muscle fibres, and named for their intimate positional or 'satellite' relationship. They have capabilities for self-renewal and for giving rise to multiple lineages in a stem cell-like function;
- "embryonic stem cells": pluripotent stem cells derived from the inner cell mass of an early stage embryo known as a blastocyst. They are able to differentiate into all derivatives of the three primary germ layers: ectoderm, endoderm, and mesoderm, thus producing each of the more than 220 cell types in the adult body;
- "tumor cells": cells that divide more than they should or do not die when they should. These cells create abnormal mass of tissue called tumors and are caused by mutations that in turn modify the growth, differenciation and death of the cells. These mutations can also modify angiogenesis once the transformed cell has migrated across the basement membrane;
- "plurality": at least two;
- "electromagnetic field": a region encompassing all of the space which exerts a force on particles (that possess the property of electric charge, and is in turn affected by the presence and motion of those particles);
- "magnetic field": a region of space in which there exists a magnetic state associated with forces;
- "electric field": a region of space where an electric state exists capable of exerting forces;
- "mechanical stimulation": the stimulation applied on the cells by the action of means, which have been previously excited by the reception of a external field;
- "modulable": variation in amplitude and frequence of the stresses, as well as the time of exposure;
- "external field generator": mechanism which generates or produces an external field, such as for example an electromagnetic, magnetic, electric, acoustic, ultrasounds;
- "cell by-products": all products capable of being for example synthesised, produced, secreted by a cell.

A first object of the invention is a cell construct comprising cells in contact with at least one basement membrane and/or at least one stroma, wherein said basement membrane or stroma contains means for applying mechanical stimulation on said cells. Preferably, the cells are retained onto the basement membrane or embbeded within the basement membrane, and the basement membrane includes beads inserted within the membrane. Moreover, the cell construct may include a stroma, on which the basement membrane may rest.

According to a particular embodiment, the basement membrane of the present invention comprises mucopolysaccharides and/or proteins. Preferably, the mucopolysaccharides (or glycosaminoglycans which are long unbranched polysaccharides comprising a repeating disaccharide unit) are chosen from the group, but not limited to, glucuronic acid, iduronic acid, hyaluronic acid, galactose, galactosamine, glucosamine. The proteins may be fibrous protein and may be chosen from the group, but not limited to collagen and fibronectin.

According to a preferred embodiment, the stroma of the present invention accompanies benign or malignant tumors and may comprise long-term culture initiating cells.

According to yet another particular embodiment, the cell construct of the present invention comprises any living cells from bacterial origin, plant origin and/or animal origin including human. Preferably, the cells comprise non-cancerous, pre-cancerous, cancerous cells and/or tissues, and wherein said cells are stem cells and preferably embryonic or adult, hematopoietic or non hematopoietic stem cells.

According to a particular embodiment, the means of the present invention used for applying mechanical stimulation on the cells are made from a material capable of receiving an external field and applying a mechanical tension. The capability of the means to emit a mechanical tension brings forward the possibility of applying a mechanical stimulation to the cells. This stimulation is preferably done in a "wireless" fashion, that is without direct contact between the means and the cells. Preferably, the material of the invention is a plurality of metallic beads preferably micro and/or nano particles, said particles being susceptible to the external field. The metallic beads can be ferric or ferrimagnetic particles.

According to yet another preferred embodiment, the external field of the present invention is chosen from the group comprising electromagnetic, magnetic and/or electric fields. The external field is modulable, meaning that it may be highly precise and controlled, thus resulting into a modulable, precise and controlled mechanical stimulation affecting the cells partly or completely.

According to another embodiment, the cell construct is a three-dimensional cell construct which preferably comprises cells, a basement membrane, a stroma and beads inserted into said basement membrane and/or stroma.

A second object of the present invention is a process for the manufacture of a cell construct as defined previously, said process of manufacture comprising:
- inserting means into a basement membrane and/or into a stroma,
- contacting cells with said basement membrane and/or stroma.

A third object of the present invention is a device for stimulating cells comprising:
- at least one cell construct as defined previously,
- a support system upon which said cell construct can be layed upon,
- a external field generator.

According to a preferred embodiment, this device is placed in or proximal of tumor cells of an animal, especially a human in a manner where the device is capable of changing the phenotype of tumor cells. Preferably, this device can be used *ex vivo* or *in vivo.* When it is used in vivo the device is said to be implantable into a subject.

A fourth object of the invention is a process for stimulating cells comprising applying an external field on the cell construct as defined previously resulting in changing the cell phenotype, inducing or increasing cell differenciation or production of cell by-products. Preferably, said stimulation resulting in inducing or increasing the production of cell by-products, permits the further recovery of cell by-products.

A fifth object of the present invention is an implant comprising:
- whole or part of the cell construct as defined previously, or
- means as defined previously embedded into any injectable vector,
said implant being subsequently inserted in or proximal of a targeted cell tissue in a manner where the implant is capable of changing the phenotype of tumor cells.
Process for stimulating cells in vivo comprising:
- inserting an implant according to the invention in or proximal of a targeted tumorous stroma of a subject suffering from a benign or malignant tumour, and
- applying an external field on said implant resulting in mechanical stimulation of the surrounding tumor cells.

Another object of the present invention is a process for stimulating cells *in vivo* comprising:
- inserting an implant as defined above in or proximal of a targeted tumorous stroma of a subject suffering from a benign or malignant tumour in a manner where the implant is capable of changing the phenotype of the tumor cells, and
- applying an external field on said implant resulting in mechanical stimulation of the tumor cells.

Other objects, advantages and features of the present invention will become more apparent upon reading of the following non restrictive description of preferred embodiments thereof, given by way of non-limiting examples, with reference to the accompanying drawings.
Figure 1 is a representation of a cell construct according to the invention.
Figure 2 is a magnified section of a cell construct according to the invention.
Figure 3 is a representation of a subject and the tumoral region of insertion of an implant according to the invention.
Figure 4 is a magnified representation of a tumor.

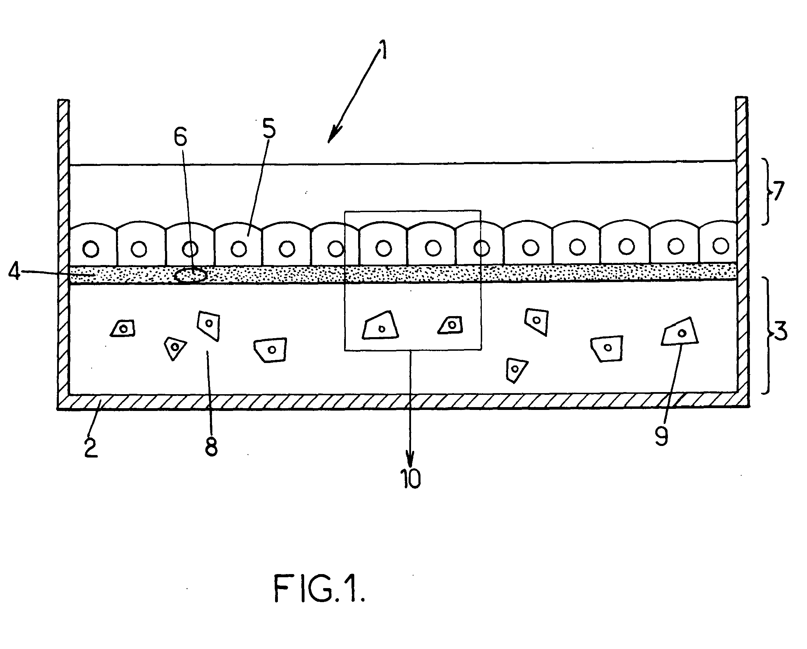

Figure 1, shows a cell construct (1) that is layed upon a support system (2) and which comprises a stroma (3), a basement membrane (4) and cells (5). The basement membrane futher comprises a plurality of beads (6) for applying the mechanical stimulation onto the cells. The culture medium (7), the extracellular matrix (8) and the stromal cells (9) are also represented on the figure.

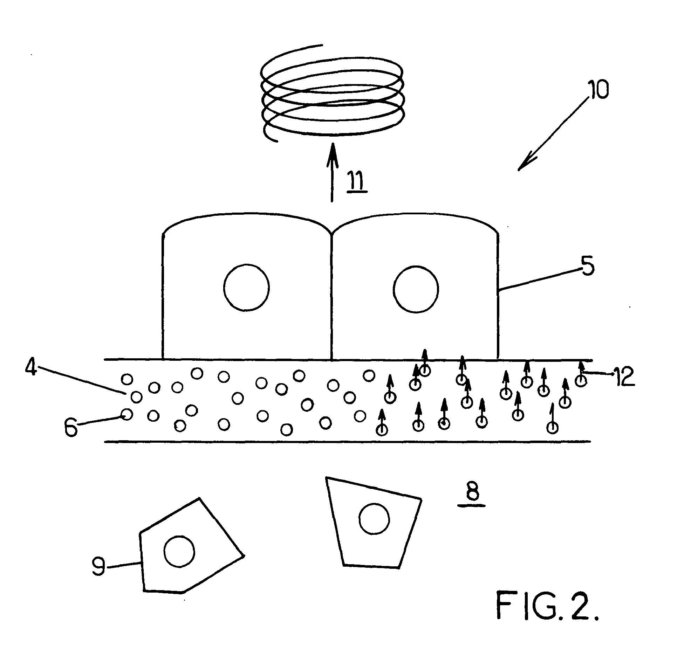

Figure 2, shows the magnification (10) of a section of the cell construct (1) to which is applied an external field (11). In particular, upon receiving an external field (11), which can be for example, electromagnetic, magnetic and/or electric, the beads (6) will then emit a mechanical tension (12) onto the cells (5).

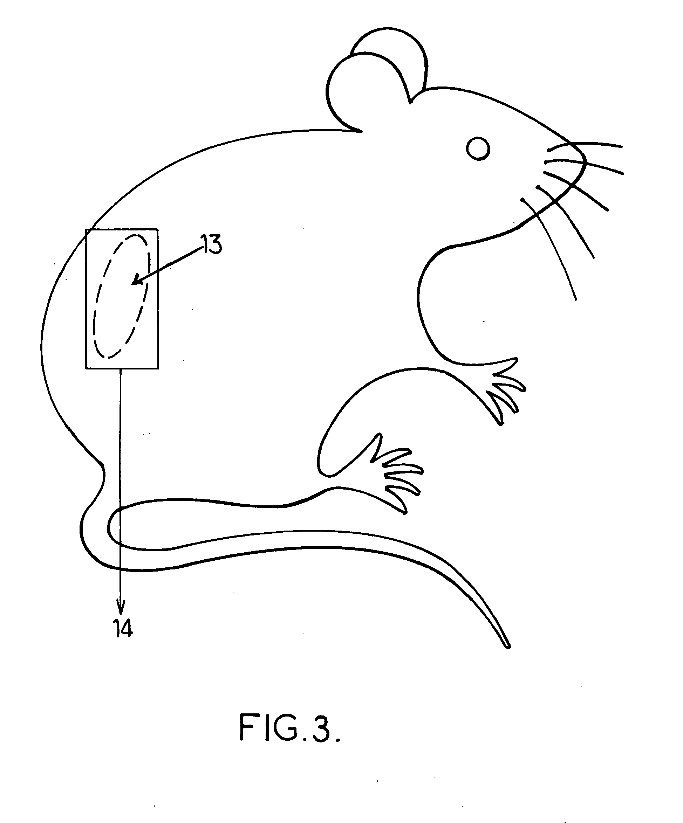

Figure 3 represents a subject into which an implant according to the invention may be inserted. In particular, the implant is to be inserted in or proximal of a tumoral region (13) of a subject.

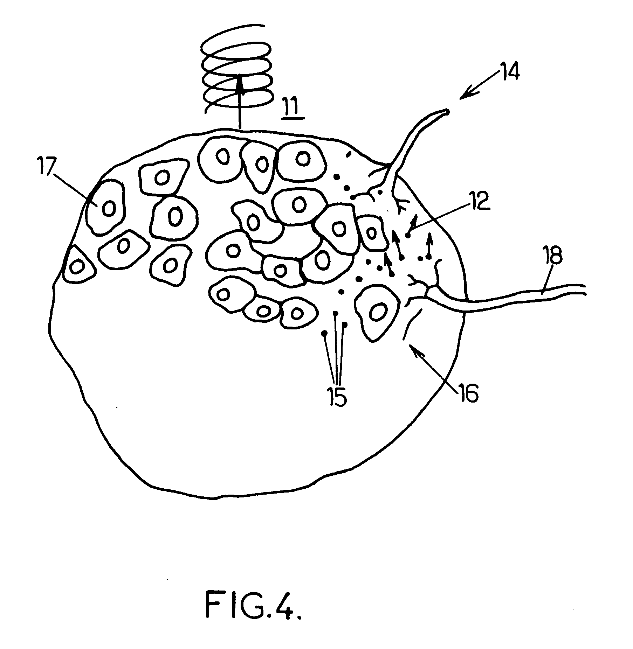

Figure 4 depicts a magnified representation (14) of the insertion of an implant (15) into the tumorous stroma of a subject (16). Upon receiving an external field (11), which can be for example electromagnetic, magnetic, electric, the implant (15), via its means, the whole cell construct or part of the cell construct will then emit the mechanical tension (12) onto the tumor cells (17). Vessels (18) are also respresented on the figure.

### EXAMPLES:

### EXAMPLE 1: Fabrication of a cell construct.

A cell construct is prepared according to general principles of cell culture relating to the particulate cell type to be cultured and includes:
- a stroma consisting in cells which are known to be used in support of the growth of animal and human stem cells ;
- a basement membrane consisting in a layer of collagen and fibronectine;
- human tumor cells; and
- nano ferric particles inserted within the basement membrane.

### EXAMPLE 2: Transformation of breast cancer cells/tissue into normal cells/tissue.

An external electromagnetic field of is applied *in vitro* to a cell construct containing nano ferric particles. The nano ferric particles will then emit a mechanical tension from 500 to 1500 Pa towards the human breast cancer cell/tissue and transform the cell phenotype from cancerous to a non-cancerous phenotype.

### EXEMPLE 3: Stimulation of cells in vivo.

The in vivo stimulation of cells is done by:
- injecting tumor cells into a mouse;
- inserting an implant into the tumorous stroma of a mouse;
- applying an external electromagnetic field of on said implant resulting into the mechanical stimulation of the tumor cells.

Although the present invention has been described hereinabove by way of preferred embodiments thereof, it can be modified, without departing from the spirit and nature of the subject invention as defined in the appended claims.

## Claims

1. Cell construct comprising cells in contact with at least one basement membrane and/or at least one stroma, wherein said basement membrane or stroma contains means for applying mechanical stimulation on said cells.

2. Cell construct according to Claim **1,** wherein said basement membrane comprises mucopolysaccharides and/or proteins.

3. Cell construct according to Claims **1 or 2,** wherein said stroma accompanies benign or malignant tumors.

4. Cell construct according to any one of Claims **1 to 3,** wherein said cells comprise any living cells from bacterial origin, plant origin and/or animal origin, including human.

5. Cell construct according to any one of Claims **1 to 4,** wherein said cells comprise non-cancerous, pre-cancerous, cancerous cells and/or tissues, and wherein said cells are stem cells and preferably embryonic or adult, hematopoietic or non hematopoietic stem cell.

6. Cell construct according to any one of Claims **1 to 5,** wherein said means for applying mechanical stimulation on the cells is a material capable of receiving an external field and applying a mechanical tension.

7. Cell construct according to Claim **6,** wherein said material is a plurality of metallic beads preferably micro and/or nano particles, said particles being susceptible to the external field.

8. Cell construct according to Claims **6 or 7,** wherein said external field is chosen from the group comprising electromagnetic, magnetic and/or electric fields.

9. Cell construct according to any one of Claims **6 to 8,** wherein said external field is modulable.

10. Cell construct according to any one of Claims **1 to 9,** wherein the cell construct is a three-dimensional cell construct.

11. Cell construct according to any one of Claims **1 to 10** comprising cells, a basement membrane, a stroma and beads inserted into said basement membrane and/or stroma.

12. Process for the manufacture of a cell construct as defined in any one of claims **1 to 11** comprising:
- inserting means into a basement membrane and/or into a stroma,
- contacting cells with said basement membrane and/or stroma.

13. Device for stimulating cells comprising:
- at least one cell construct as defined in any one of Claims **1 to 11,**
- a support system upon which said cell construct can be layed upon,
- an external field generator.

14. Process for stimulating cells comprising applying an external field on the cell construct as defined in any one of Claims **1 to 11** resulting in changing the cell phenotype, inducing or increasing cell differenciation or production of cell by-products.

15. Process according to Claim **14,** wherein the stimulation results in inducing or increasing the production of cell by-products, wherein cell by-products are further recovered.

16. Implant comprising:
- whole or part of the cell construct according to any one of claims **1 to 11,** or
- means according to any one of claims **6 or 7,** embedded into any injectable vector,
said implant being subsequently inserted in or proximal of a targeted cell tissue in a manner where the implant is capable of changing the phenotype of tumor cells..

17. Process for stimulating cells *in vivo* comprising:
- inserting an implant according to claim **16** in or proximal of a targeted tumorous stroma of a subject suffering from a benign or malignant tumour in a manner where the implant is capable of changing the phenotype of the tumor cells, and
- applying an external field on said implant resulting in mechanical stimulation of the tumor cells.
